# EUROPEAN PATENT SPECIFICATION

(11) **EP 1 913 905 B1**
(45) Date of publication and mention of the grant of the patent: **03.10.2012**
(21) Application number: 06782268.4
(22) Date of filing: 03.08.2006
(51) Int. Cl.: A61F 5/00

(54) **BALLOON TO BE PLACED AND LEFT IN STOMACH**
BALLON ZUR PLATZIERUNG UND ZUM VERBLEIB IM MAGEN
BALLONNET DESTINÉ À ÊTRE PLACÉ ET LAISSÉ DANS L'ESTOMAC

(30) Priority: 11.08.2005 JP 2005232882
(43) Date of publication of application: 23.04.2008
(73) Proprietor: OLYMPUS MEDICAL SYSTEMS CORP., Tokyo 151-0072 (JP)
(72) Inventor: KAJI, Kunihide, Hachioji-shi, Tokyo 1920023 (JP); MIKKAICHI, Takayasu, Fuchu-shi, Tokyo 1830033 (JP)
(74) Representative: von Hellfeld, Axel
(86) International application number: PCT/JP2006/315408
(87) International publication number: WO 2007/018122

(56) References cited:
- EP-A- 0 137 878
- WO-A1-2004/014237
- DE-A1- 10 158 785
- FR-A- 2 861 288
- JP-A- 63 277 063

## Description

### BACKGROUND OF THE INVENTION

The present invention relates to an intragastric balloon that is left in a stomach for an extended period in order to perform weight loss treatment on morbidly obese patients.

### Description of Related Art

Conventionally, two methods are mainly considered for weight loss measures for morbidly obese patients, namely, a surgical treatment method and an endoscopic treatment method. The method which is selected and the treatment which is performed are determined in accordance with the patient.

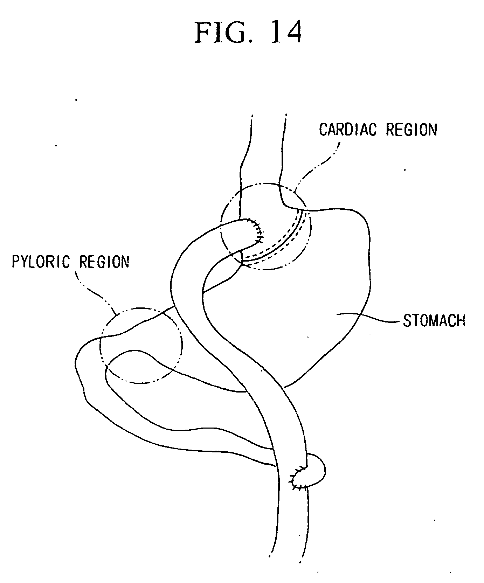

A surgical treatment method is a method in which a stomach is made smaller, for example, by surgery and in which, as is shown in FIG 14, the stomach is closed in the vicinity of the cardiac region and the digestive tract below the duodenum is then directly connected to this point. According to this method, the stomach capacity is restricted and it is possible to limit the amount of food that is ingested. In addition, a feeling that the stomach is full can be obtained with the intake of only a small amount of food. Furthermore, in the case of this latter, because the length of the digestive tract through which food passes is shortened, nutrient absorption is impeded, and weight loss can be effectively achieved.

However, in this surgical treatment method, the burden on the patient is considerable, and because, moreover, the stomach is excised and then rejoined, it is not possible for the original state to be restored, so that this method does not allow for the operation to be performed again. Because of this, this treatment is not one that can be undertaken easily by just anybody.

In contrast, in an endoscopic treatment method, surgery is not required so that the burden on the patient is small. Moreover, this method has the advantage that the operation is able to be performed again so that, currently, this method is one attracting widespread attention.

This endoscopic treatment method is one in which, using an endoscope, a balloon (i.e., a gastric balloon) is inserted in a deflated state into a stomach and, after this insertion, the balloon is inflated inside the stomach and is then left in an inflated state inside the stomach (see, for example, Patent Document 1: United States Patent (US-P) No. 4694827 and Patent Document 2: United States Patent (US-P) No. 5084061).

According to this method, because the balloon is left inside the stomach at all times, it is possible to limit the amount of food consumed in a single meal. As a result, weight loss can be effectively achieved.

In particular, because this endoscopic treatment method makes it possible to easily extract the balloon from inside the stomach using an endoscope device, this method is one in which the operation is able to be performed a number of times, as is mentioned above. Because of this, unlike in a surgical treatment method, this method has the advantage that anybody can easily undergo treatment.

However, in the conventional endoscopic treatment method disclosed in the aforementioned Patent Documents 1 and 2, the following problems have existed.

Namely, conventionally, when a deflated balloon is inserted into a stomach and then inflated, the balloon is inflated by pouring pure water or physiological saline solution or the like into it. Because of this, the inflated balloon becomes heavy and when the patient stands up, it is common for the balloon to be left blocking the pyloric region. Because of this, it becomes difficult for the stomach contents to pass to the digestive tract side and the possibility arises that a passage blockage will be created. As a result, the patient tends to easily become nauseous and feel uncomfortable, and it has led to harmful effects on the body.

DE 101 58 785 discloses a stomach implant having a generally spherical main body and first and second cylindrical ends adapted to be fixed to an oesophagus and a small intestine of a patient. The first and second cylindrical ends have fixing means adapted to fix the cylindrical ends to the oesophagus and the small intestine. The first and second cylindrical ends communicate with the spherical main body, wherein the volume of the spherical main body corresponds to the desired volume of the patient's stomach. The implant has the effect of connecting the patient's oesophagus and small intestine such that the stomach and the duodenum are completely bypassed.

EP 0 137 878, which is regarded as the closest prior art, discloses a torus-shaped stomach insert comprising a flexible, inflatable balloon which is free-floating and unattached in the stomach. The insert comprises a passage therethrough adapted for passing solids and liquids. When the insert is inserted in to the stomach, the insert is positioned within the stomach using a stomach tube, and the insert is then inflated. The preamble of appended claim 1 is based on this document.

FR 2 861 288 discloses an spherical expandable intragastric balloon having a pocket adapted to be inflated with a gas and comprising a ballast adapted to increase the weight of the balloon in order to improve its position within the stomach. Because intragastric balloons filled with gas have the tendency to position themselves in the upper part of the stomach, thereby impairing the passage of food from the oesophagus into the stomach and leading to discomfort for the patient, the ballast means is provided. The ballast means may comprise dense solid bodies disposed inside the balloon, or a single ball comprised of a dense material, wherein the ballast means can freely move inside the balloon. If several solid bodies are used, the bodies should be attached to one another, for example, magnetically, or via flexible or rigid lines, so as to limit the movement of the solid bodies with respect to each other. Attaching the bodies results in a reduction of shock or feeling the movement of the small bodies in the stomach. Alternatively, the ballast means can comprise sponges disposed on the outer wall of the balloon, wherein the sponges absorb a small amount of liquid disposed in the balloon to create a ballast weight. Still further, the ballast means may comprise a sheath disposed on a portion of the balloon wall, wherein the sheath has small dense bodies disposed therein.

### SUMMARY OF THE INVENTION

The present invention was conceived in view of the above described problems in the conventional technology, and it is an object thereof to provide an intragastric balloon that, when left inside a stomach, has no possibility of blocking the passage of stomach contents, and that enables weight loss to be effectively achieved.

In order to achieve the above described object, an intragastric balloon according to claim 1 is provided.

The present invention is an intragastric balloon that is left in an inflated state inside a stomach and includes: a balloon main body that is capable of being inflated to a predetermined size; and at least one or more internal passages that are formed in the balloon main body so as to penetrate the balloon main body, and that allow contents of the stomach to be passed.

In the intragastric balloon of the present invention, when the balloon main body is left in a stomach and inflated to a predetermined size, because an internal passages is formed in the balloon main body itself, even if the balloon main body temporarily blocks off the pyloric region, the stomach contents do not become jammed and create a passage obstruction. Namely, because the stomach contents are able to pass through the internal passage which penetrates the balloon main body and move to the opposite side of the balloon main body, they can flow into the digestive tract below the pyloric region, and it is possible to prevent passage obstacles being created. Accordingly, the patient feels no nausea or sense of discomfort or the like.

Moreover, because the balloon main body is left in the stomach, the satiety center is stimulated by only a small calorific intake. Therefore, a patient is able to restrict the amount of food ingested in a single meal. As a result, weight loss can be effectively achieved.

As has been described above, according to the intragastric balloon of the present invention, there is no possibility of the passage of stomach contents being blocked, and it is possible to effectively achieve weight loss.

In the present invention, it is also possible for the balloon main body to be formed in an elliptical shape, and for the internal passage to be formed extending in the axial direction of the balloon main body.

In the intragastric balloon of the present invention, because the balloon main body is formed in an elliptical shape, when the balloon main body is left inside the stomach the orientation thereof is naturally stabilized following the internal configuration of the stomach. Namely, the orientation is stabilized in a state in which the transverse axis is aligned in a direction connecting the lesser curvature to the greater curvature inside the stomach, and in which the longitudinal axis is aligned in a direction connecting a stomach fundus portion or cardiac region to the pyloric region.

Here, because the internal passage is formed extending along the longitudinal axis, which is a direction connecting the cardiac region where food is brought into the stomach to the pyloric region where the food is carried to the intestinal duodenum and the small intestine and the like, it is possible to more reliably eliminate any obstruction to the passage of the stomach contents.

In the present invention, the balloon is provided with an internal passage controlling portion that positions the balloon main body such that the internal passage is aligned in a direction connecting the cardiac region to the pyloric region.

In the intragastric balloon of the present invention, when the balloon main body is left inside a stomach, because the internal passage controlling portion positions the balloon main body such that the internal passage is aligned in a direction connecting the cardiac region where food is brought into the stomach to the pyloric region where the food is carried to the intestinal duodenum and the small intestine and the like, it is possible to more reliably eliminate any obstruction to the passage of the stomach contents.

In the present invention, the internal passage controlling portion adjusts the position of the center of gravity of the balloon main body such that an aperture portion on one end side of the internal passage is heavier than an aperture portion on another end side of the internal passage.

In the intragastric balloon of the present invention, when the balloon main body is left inside a stomach, because the position of the center of gravity thereof is adjusted by the internal passage controlling portion, the attitude thereof changes inside the stomach due to gravity and the intragastric balloon is stabilized with the aperture portion on the one end side of the internal passage positioned on the pyloric region side. As a result, the direction of the internal passage is controlled so as to connect the cardiac region with the pyloric region.

In this manner, by using gravity, the balloon main body can be positioned in a suitable orientation, and any obstruction to the passage of the stomach contents can be eliminated.

In the present invention, it is also possible for the balloon main body and the internal passage to each be formed from an optically transparent material.

In the intragastric balloon of the present invention, because the balloon main body and the internal passage are formed from an optically transparent material, it is possible to observe the interior of the stomach using an endoscope device with the balloon main body in an inflated state. Namely, when the endoscope insertion portion of an endoscope device is inserted into the interior of the stomach, the interior of the stomach can be observed on the other side of the balloon main body, and it is possible to confirm whether or not an ulcer or the like is present on the stomach wall. In addition, it is also possible to insert the endoscope insertion portion into the internal passage and observe the interior of the stomach. Accordingly, it is possible to obtain an improvement in usability such as through observations being made easier.

According to the intragastric balloon of the present invention, there is no possibility of blocking the passage of stomach contents, and it is possible to effectively achieve weight loss.

### BRIEF DESCRIPTION OF THE DRAWINGS

FIG. 1 is view explaining a first example useful for understanding the present invention, and shows an intragastric balloon that has been left inside a stomach in an inflated state.
FIG 2 is a view showing the intragastric balloon shown in FIG 1.
FIG 3 is a cross-sectional view of an insertion aperture and the periphery of a valve that are provided in a balloon body of the intragastric balloon shown in FIG 1.
FIG. 4A is a view showing a state in which the intragastric balloon shown in FIG. 1 is left inside a stomach.
FIG 4B is a view showing a variant example in which the orientation of the internal passage is different from that in FIG. 4A.
FIG 5 is a view showing a first embodiment of the intragastric balloon according to the present invention.
FIG. 6 is a view of the intragastric balloon shown in FIG 5 as seen from an aperture portion on one end of an internal passage.
FIG 7 is a view showing a state in which the intragastric balloon shown in FIG 5 is left inside a stomach.
FIG. 8 is a view showing another example of the first embodiment of the intragastric balloon according to the present invention.
FIG. 9 is a view showing a second example useful for understanding the present invention.
FIG. 10 is a view showing another example useful for understanding the present invention.
FIG 11 is a view showing an example useful for understanding the present invention, and is a side view of an intragastric balloon that is formed by combining small balloons into a balloon main body.
FIG 12 is a cross-sectional view taken along the line A-A in FIG 11.
FIG 13 is a view showing an example useful for understanding the present invention, and is a view showing an intragastric balloon in which a balloon main body is formed by a cylindrical balloon inside which is formed an internal passage, and ring-shaped balloons that encircle an outer circumference of the cylindrical balloon.
FIG 14 is a view showing an example of a conventional surgical treatment method for morbid obesity, and shows a state of a stomach after surgery.

### DETAILED DESCRIPTION OF THE INVENTION

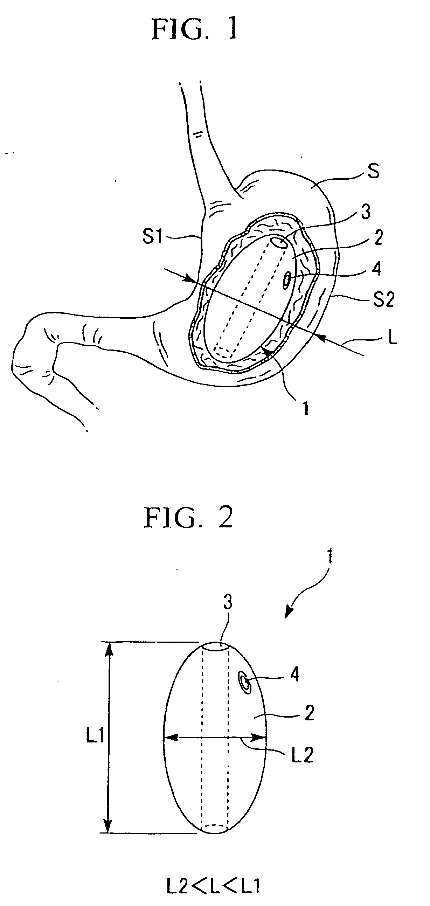

A first example of intragastric balloon useful for understanding the present invention will now be described with reference made to FIG 1 through FIG 4A.

As is shown in FIG 1 and FIG. 2, an intragastric balloon 1 of the present example is left in an inflated state inside a stomach S, and is provided with a balloon body 2 that is capable of being inflated to a predetermined size, and at least one internal passage 3 that penetrates through the balloon main body 2, and that allows contents of the stomach S to pass through it.

Note that an example is shown in which one internal passage 3 is formed.

The above described balloon main body 2 has expandability to allow it to freely expand and contract, and is also formed from an optically transparent material. After it has been inserted in a deflated state into a stomach S using an endoscope device (not shown), the balloon main body 2 is then inflated inside the stomach S as a result of pure water or physiological saline solution or the like then being supplied via a dedicated insertion tube X that has been inserted into the channel of the endoscope device. Moreover, the balloon main body 2 is formed such that when inflated, it has an elliptical shape.

In addition, the balloon main body 2 is formed such that a longitudinal axis L1 is larger than, and such that a transverse axis L2 is shorter than a distance L connecting a lesser curvature S 1 and a greater curvature S2 inside a stomach S.

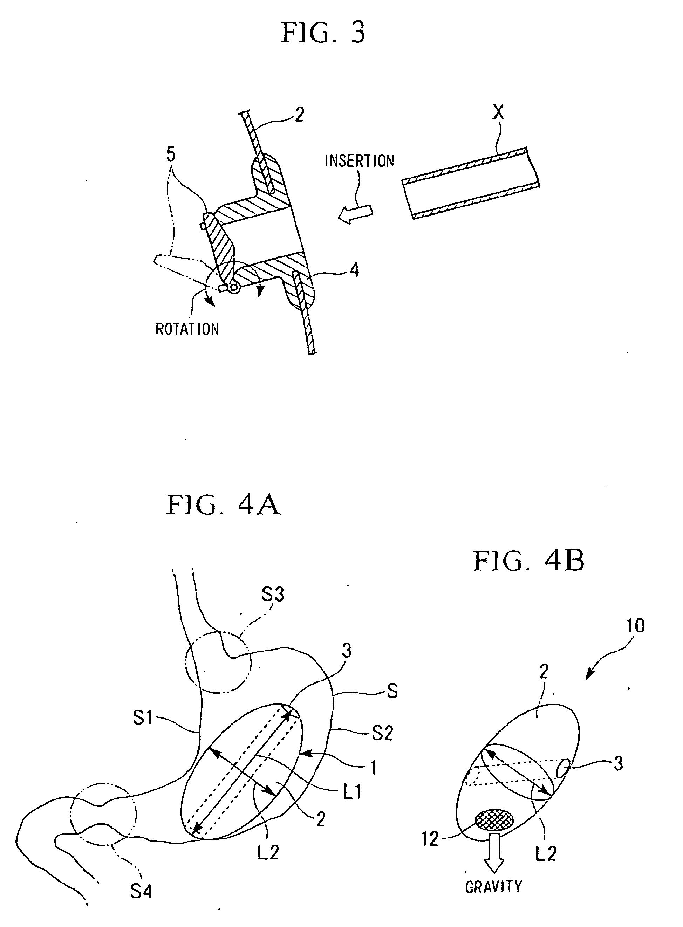

As is shown in FIG 3, an insertion aperture 4 into which the aforementioned insertion tube X is inserted is provided in an outer circumferential surface of the balloon main body 2. A flap valve type of valve 5 that automatically closes the aperture of the insertion aperture 4 is provided in this insertion aperture 4. This valve 5 is rotatably connected by a pin to the insertion aperture 4, and normally blocks of the aperture by means of a spring (not shown).

Namely, when the insertion tube X has been inserted into the insertion aperture 4, the valve 5 is pushed by the distal end of the insertion tube X and is rotated such that the closed state of the aperture of the insertion aperture 4 is released. In addition, when the insertion tube is withdrawn from the insertion aperture 4, the valve 5 is urged by the spring and automatically closes the aperture of the insertion aperture 4.

The aforementioned internal passage 3 is formed at a size that enables the endoscope insertion portion to be inserted therethrough, and extends along the longitudinal axis L1 of the balloon main body 2. Moreover, in the same way as the balloon main body 2, this internal passage 3 is also optically transparent.

Next, a case in which treatment of a morbidly obese patient is performed using the intragastric balloon 1 which is constructed in this manner will be described.

Firstly, using an endoscope device (not shown), the balloon main body 2 is inserted into the stomach S in a deflated state. After the balloon main body 2 has been inserted, the insertion tube X is inserted into the channel of the endoscope device and, while the endoscopic images are being monitored, as is shown in FIG. 3, the insertion tube X is pushed through the insertion aperture 4 of the balloon main body 2. At this time, because the valve 5 is opened as a result of the insertion tube X being pushed through the insertion aperture 4, the interior of the balloon main body 2 and the insertion tube X are in a state of mutual communication.

After the insertion tube X has been inserted, pure water or physiological saline solution or the like is then supplied to the interior of the balloon main body 2 via the insertion tube X, and the balloon main body 2 is inflated to a predetermined size. After the balloon main body 2 has been inflated, the insertion tube X is withdrawn from the insertion aperture 4, and the endoscope device including the insertion tube X is extracted from inside the patient. As a result, as is shown in FIG. 1, the intragastric balloon 1 is left inside the stomach S of the patient in an inflated state. Note that by extracting the insertion tube X, because the valve 5 of the balloon main body 2 is edged by the spring and automatically closes the aperture of the insertion aperture 4, the interior of the balloon main body 2 is not in communication with the outside thereof via the insertion aperture 4.

In particular, because the balloon main body 2 is formed in an elliptical shape when inflated, as is shown in FIG 4A, as the balloon main body 2 is inflated, the orientation of the balloon main body 2 is naturally stabilized following the internal configuration of the stomach S. Namely, the orientation is stabilized in a state in which the transverse axis L2 faces in a direction connecting the lesser curvature S1 to the greater curvature S2 inside the stomach S, and in which the longitudinal axis L1 faces in a direction connecting a stomach fundus portion or cardiac region S3 to a pyloric region S4. At this time, because the internal passage 3 is formed extending along the longitudinal axis L1 of the balloon main body 2, a path for food is formed in a direction connecting the cardiac region S3, where food is brought in, to the pyloric region S4, where the food is carried to the intestinal duodenum and the small intestine and the like.

Accordingly, even if the balloon main body 2 is temporarily left inside the stomach S in a state in which it is blocking the pyloric region S4, because the contents of the stomach S pass along the internal passage 3 and flow into the pyloric region S4, it is possible to prevent any obstruction to the passage of the contents of the stomach S being generated. Accordingly, the patient feels no nausea or sense of discomfort or the like.

In particular, depending on the shape of the balloon main body 2, because the orientation of the internal passage 3 is controlled, it is possible to reliably allow the contents to pass through and prevent any obstruction to their passage.

Moreover, because the balloon main body 2 is normally left inside the stomach S, the satiety center in the stomach fundus portion is stimulated by only a small calorific intake. Therefore, a patient is able to restrict the amount of food ingested in a single meal. As a result, weight loss can be effectively achieved.

Moreover, because the balloon main body 2 and the internal passage 3 are formed from an optically transparent material, it is possible to observe the interior of the stomach S using an endoscope device with the balloon main body 2 in an inflated state. Namely, when the endoscope insertion portion of an endoscope device is inserted into the interior of the stomach S, the interior of the stomach S can be observed on the other side of the balloon main body 2, and it is possible to confirm whether or not an ulcer or the like is present on the stomach wall. In addition, it is also possible to insert the endoscope insertion portion into the internal passage 3 and confirm the interior of the stomach S.

In the conventional treatment of obesity using balloons, a number of signs of ulcers and the like can be seen, however, in these cases, it is necessary to first deflate the balloon and then extract it from inside the stomach, and to then perform an endoscopic examination, which is extremely laborious. However, by making the balloon main body 2 transparent, this task is rendered unnecessary. Furthermore, by using a gas such as air or the like instead of a liquid (i.e., such as physiological saline solution or the like) as the medium to inflate the balloon, it is possible to eliminate any lens effect caused by such a medium. Accordingly, it is possible to obtain an improvement in usability such as through observations being made easier.

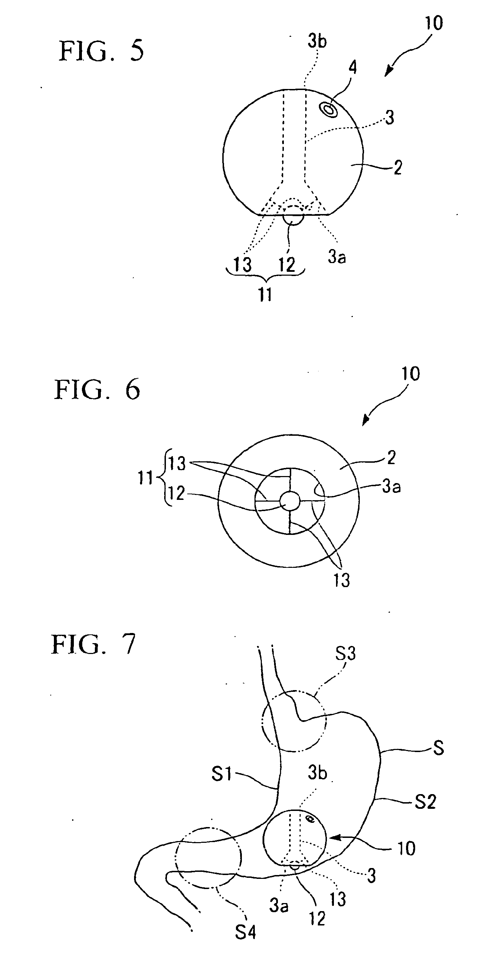

Next, a first embodiment of the intragastric balloon of the present invention will be described with reference made to FIG 5 through FIG 7. Note that in the first embodiment, component elements that are the same as those in the first example are given the same symbols and a description thereof is omitted.

The first embodiment differs from the first example in that in the intragastric balloon 1 of the first example, the orientation of the internal passage 3 was controlled by the shape of the balloon main body 2, however, in the intragastric balloon 10 of the first embodiment, the orientation of the internal passage 3 is controlled by an internal passage controlling device (i.e., an internal passage controlling portion) 11.

Namely, as is shown in FIG. 5 and FIG 6, the intragastric balloon 10 of the present embodiment is provided with the aforementioned internal passage controlling device 11 that positions the balloon main body 2 such that the internal passage 3 is oriented in a direction connecting the cardiac portion S3 to the pyloric region S4 of the stomach S. This internal passage controlling device 11 adjusts the position of the center of gravity of the balloon main body 2 such that an aperture portion 3a on one end side of the internal passage 3 is heavier than an aperture portion 3b on the other end side thereof.

Moreover, the balloon main body 2 of the present embodiment is formed in a spherical shape instead of an elliptical shape. In addition, the internal passage 3 is formed so as to become wider partway along the passage such that the aperture surface area of the one end side aperture portion 3a is greater than that of the other end side aperture portion 3b.

A weight portion 12 is provided substantially in the center of the one end side aperture portion 3a. This weight portion 12 is firmly supported on the balloon main body 2, for example, by four wires 13 such that it does not shift in position from the aforementioned substantially center position. The balance of the center of gravity of the balloon main body 2 is changed by means of this weight portion 12, such that the center of gravity position is offset towards the one end side aperture portion 3a.

Namely, this weight portion 12 and the wires 13 constitute the above described internal passage controlling device 11.

Furthermore, if the balloon is inflated using a medium having a smaller specific gravity than that of the weight 12, the center of gravity can be more actively offset, and control of the position of the internal passage 3 is more easily achieved.

Next, a description will be given of when treatment is performed using the intragastric balloon 10 constructed in this manner. Note that an example in which the patient is in a standing position is described.

When the balloon main body 2 of the present embodiment is inserted into a stomach S and inflated, the attitude of the weight portion 12 changes inside the stomach S due to gravity such that the weight portion 12 is positioned on the pyloric region S4 side of the stomach S. In addition, as is shown in FIG 7, the attitude of the intragastric balloon 10 is stabilized such that the weight portion 12 and the one end side aperture portion 3a of the internal passage 3 are positioned on the pyloric region S4 side, and such that the other end side aperture portion 3b of the internal passage 3 is positioned on the cardiac region S3 side. As a result, in the same way as in the first example, the direction of the internal passage 3 is controlled so as to connect the cardiac region S3 with the pyloric region S4.

Accordingly, the contents of the stomach S can be reliably carried through the internal passage 3 to the intestinal duodenum and the small intestine and the like, and it is possible to prevent any obstruction to the passage being created. In particular, because the aperture surface area of the one end side aperture portion 3a is greater than that of the other end side aperture portion 3b, the contents pass more reliably towards the pyloric region S4.

As has been described above, because the internal passage control device 11 is able to control the position of the center of gravity of the balloon main body 2 even when the balloon main body 2 has a spherical shape, it is possible to set the direction of the internal passage 3 to a suitable direction using gravity, and reliably eliminate any passage obstruction.

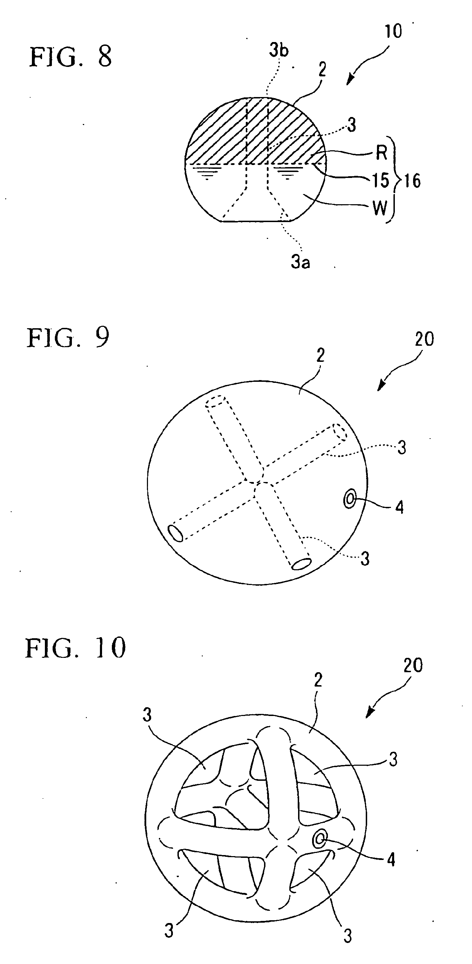

Note that in the above described first embodiment, the balance of the center of gravity of the balloon main body 2 is changed using the weight portion 12, however, the present invention is not limited to using the weight portion 12. For example, as is shown in FIG 8, it is also possible to provide a partitioning plate 15 that partitions the one end side aperture portion 3a side of the internal passage 3 from the other end side aperture portion 3b side thereof inside the balloon main body 2, and pour physiological saline solution W into the balloon main body 2 on the one end side aperture portion 3a side, and also inject air R into the balloon main body 2 on the other end side aperture portion 3b side. By doing this, the one end side aperture portion 3a side into which the physiological saline solution W has been poured becomes heavier. As a result, in the same way as when the weight portion 12 is used, the position of the center of gravity of the balloon main body 2 can be offset to the one end side aperture portion 3a side. Accordingly, the same operating effects can be achieved. Note that in this case, the partitioning plate 15, the physiological saline solution W and the air R form an internal passage control device (i.e., an internal passage control portion) 16.

Moreover, in the above described first embodiment, the shape of the balloon main body 2 was a spherical shape, however, it is also possible for it to be formed as an elliptical shape in the same way as in the first example. By forming the balloon main body 2 as an elliptical shape, it is possible to more fully stabilize the attitude of the balloon main body 2 using the effects of both the shape of the balloon main body 2 and the internal passage controlling device 11, and the orientation of the internal passage 3 can be controlled more reliably. Note that, as is shown in FIG. 4B, the internal passage 3 at this time may also be provided so as to penetrate a plane P which forms the transverse axis L2 of the elliptical balloon main body 2.

Next, a second example useful for understanding the present invention will be described with reference made to FIG. 9.

Note that in the second example, component elements that are the same as those in the first embodiment are given the same symbols and a description thereof is omitted.

The second example differs from the first example in that while a single internal passage 3 is formed in the intragastric balloon 1 of the first example, two internal passages 3 are formed in an intragastric balloon 20 of the second example.

Namely, as is shown in FIG. 9, two internal passages 3 which are mutually orthogonal to each other are formed in the balloon main body 2 of the intragastric balloon 20 of the present example.

According to the intragastric balloon 20 that is formed in this manner, when the balloon main body 2 is inflated inside the stomach S, it is not necessary to stabilize the attitude of the balloon main body 2 in order to control the orientation of the internal passage 3 as in the first example. Namely, because there are two internal passages 3, the contents of the stomach S can be made to travel in the direction of the pyloric region S4 irrespective of the attitude of the balloon main body 2.

Moreover, the number of internal passages 3 is not limited two, and maybe three or more. In particular, the greater the number of internal passages 3, the less important the attitude of the balloon main body 2 as the contents are able to pass using any of the internal passages 3. This is preferable as it makes it even more possible to prevent the occurrence of passage obstructions.

Furthermore, when a plurality of internal passages 3 are formed, as is shown in FIG 10, it is also possible to construct the balloon main body 2 by joining the internal passages 3 together so as to create a spherically-shaped basic framework. By employing such as structure, the contents of the stomach S can be made to pass even more smoothly, and it is possible to even more completely eliminate the generation of passage obstructions.

Note that the technological range of the present invention is not limited to the above described embodiments and various modifications may be made thereto insofar as they do not depart from the scope of the present invention as defined by the claims.

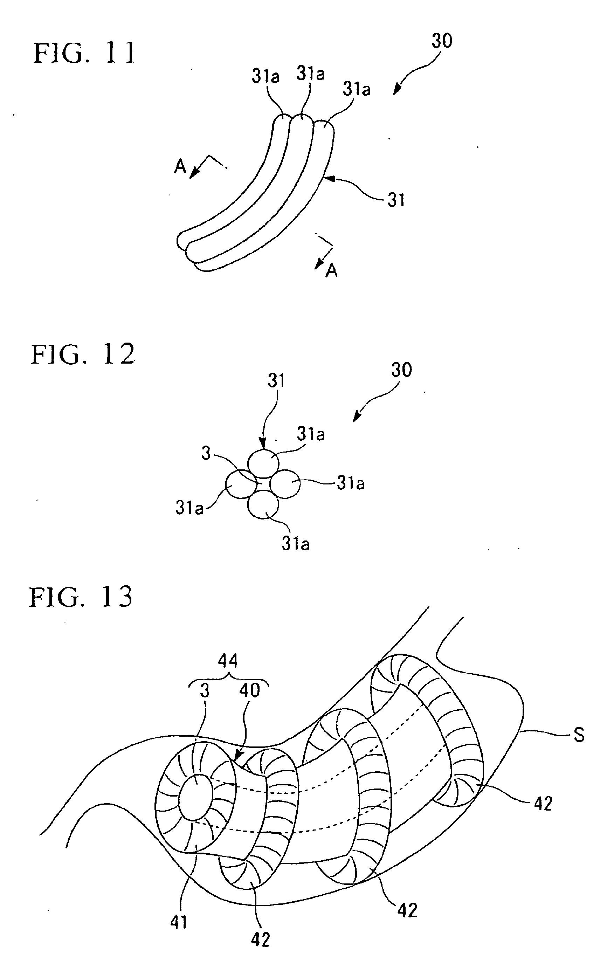

For example, in each of the above described embodiments, the balloon main body 2 is formed in an elliptical shape or spherical shape, however, the present invention is not limited to this shape and the balloon main body 2 may also be formed in a crescent shape so as to match the internal contour of the stomach S. Moreover, as is shown in FIG. 11 and FIG 12, it is also possible to construct a balloon main body 31 by combining, for example, four crescent-shaped small balloons 31a. In this case, a space enclosed by the small balloons 31a forms the internal passage 3. In the same way as in each of the above described embodiments, it is also possible to eliminate the generation of passage obstructions in an intragastric balloon 30 that is formed in this manner.

Furthermore, as is shown in FIG. 13, it is also possible to construct a balloon main body 40 from a cylindrical balloon 41 in which an internal passage 3 is formed, and from a plurality of ring-shaped balloons 42 that encircle the circumference of the cylindrical balloon 41. In the same way as in each of the above described embodiments, it is also possible to eliminate obstructions to the passage of stomach contents in an intragastric balloon 44 that is formed in this manner.

### INDUSTRIAL APPLICABILITY

The present invention can be applied to an intragastric balloon that is left for an extended period inside a stomach in order to perform weight-loss treatment for morbidly obese patient: According to the intragastric balloon of the present invention, there is no possibility of the passage of the contents of a stomach being obstructed, and it is possible to effectively achieve a weight loss effect.

## Claims

1. An intragastric balloon (10) that is adapted to be left in an inflated state inside a stomach (S) comprising:
a balloon main body (2) that is capable of being inflated with a liquid to a predetermined size; and
an internal passage (3) that is formed in the balloon main body (2) so as to penetrate the balloon main body (2), and that is adapted to allow contents of the stomach (S) to be passed therethrough
**characterized by** further comprising an internal passage controlling portion (11, 16) provided in the balloon main body (2) and being adapted to adjust the position of the center of gravity of the balloon main body (2) such that the internal passage (3) is aligned in a direction connecting the cardiac region (S3) to the pyloric region (S4).

2. The intragastric balloon according to claim 1, wherein
the balloon main body (2) is formed in an elliptical shape, and
the internal passage (3) is formed extending in the axial direction of the balloon main body (2).

3. The intragastric balloon according to claim 1, wherein
the internal passage controlling portion (11, 16) adjusts the position of the center of gravity of the balloon main body (2) such that an aperture portion (3a) on one end side of the internal passage (3) is heavier than an aperture portion (3b) on another end side of the internal passage (3).

4. The intragastric balloon according to claim 1, wherein
the balloon main body (2) and the internal passage (3) are each formed from an optically transparent material.

## Patentansprüche

1. Intragastrischer Ballon (10), der dazu eingerichtet ist, in einem aufgeblähten Zustand im Inneren eines Magens (S) zu verbleiben, mit
einem Ballonhauptkörper (2), der mit einer Flüssigkeit auf eine vorgegebene Größe aufgebläht werden kann; und
einem Innendurchlass (3), der in dem Ballonhauptkörper (2) so ausgebildet ist, dass er den Ballonhauptkörper (2) durchdringt, und der dazu eingerichtet ist, dass Inhalte des Magens (S) durch ihn hindurchgehen können,
**dadurch gekennzeichnet, dass** er ferner einen Innendurchlass-Steuerbereich (11, 16) umfasst, der im Ballonhauptkörper (2) vorgesehen ist und der die Position des Schwerpunkts des Ballonhauptkörpers (2) so einzustellen vermag, dass der Innendurchlass (3) in einer Richtung ausgerichtet ist, die den Kardialbereich (S3) mit dem pylorischen Bereich (S4) verbindet.

2. Intragastrischer Ballon nach Anspruch 1, wobei
der Ballonhauptkörper (2) in einer elliptischen Form ausgebildet ist und
der Innendurchlass (3) so ausgebildet ist, dass er sich in der axialen Richtung des Ballonhauptkörpers (2) erstreckt.

3. Intragastrischer Ballon nach Anspruch 1, wobei
der Innendurchlass-Steuerbereich (11, 16) die Position des Schwerpunkts des Ballonhauptkörpers (2) so einstellt, dass ein Öffnungsbereich (3a) an einer Endseite des Innendurchlasses (3) schwerer ist als ein Öffnungsbereich (3b) an der anderen Endseite des Innendurchlasses (3).

4. Intragastrischer Ballon nach Anspruch 1, wobei
der Ballonhauptkörper (2) und der Innendurchlass (3) beide aus einem durchsichtigen Material ausgebildet sind.

## Revendications

1. Ballonnet intragastrique (10) qui est adapté à être laissé dans un état gonflé à l'intérieur d'un estomac (S) comprenant :
un corps principal (2) de ballonnet qui est apte à être gonflé avec un liquide jusqu'à une taille prédéterminée ; et
un passage interne (3) qui est formé dans le corps principal (2) de ballonnet de façon à pénétrer le corps principal (2) de ballonnet, et qui sont adaptés à permettre qu'un contenu de l'estomac (S) soit fait passer à travers ceux-ci
**caractérisé en ce que** comprenant en outre une partie de contrôle (11, 16) de passage interne prévue dans le corps principal (2) de ballonnet et étant adaptée à ajuster la position du centre de gravité du corps principal (2) de ballonnet de manière à ce que le passage interne (3) soit aligné dans une direction reliant la région cardiaque (S3) à la région pylorique (54).

2. Ballonnet intragastrique selon la revendication 1, dans lequel le corps principal (2) de ballonnet est formé à une forme elliptique, et
le passage interne (3) est formé s'étendant dans le sens axial du corps principal (2) de ballonnet.

3. Ballonnet intragastrique selon la revendication 1, dans lequel
la partie de contrôle (11, 16) de passage interne ajuste la position du centre de gravité du corps principal (2) de ballonnet de manière à ce qu'une partie d'ouverture (3a) sur un côté d'extrémité du passage interne (3) soit plus lourde qu'une partie d'ouverture (3b) sur un autre côté d'extrémité du passage interne (3).

4. Ballonnet intragastrique selon la revendication 1, dans lequel
le corps principal (2) de ballonnet et le passage interne (3) sont chacun formés d'un matériau optiquement transparent.
